(19) Europäisches Patentamt
European Patent Office
Office européen des brevets

(11) **EP 3 203 397 B1**

(12) **EUROPEAN PATENT SPECIFICATION**

(45) Date of publication and mention
of the grant of the patent:
**01.04.2020 Bulletin 2020/14**

(51) Int Cl.:
*A61M 16/14* *(2006.01)*    *A61M 15/00* *(2006.01)*
*G16H 20/10* *(2018.01)*

(21) Application number: **16154634.6**

(22) Date of filing: **08.02.2016**

(54) **MEDICAL EVALUATION DEVICE**

MEDIZINISCHE BEURTEILUNGSVORRICHTUNG

DISPOSITIF D'ÉVALUATION MÉDICALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**09.08.2017 Bulletin 2017/32**

(73) Proprietor: **PARI Pharma GmbH
82319 Starnberg (DE)**

(72) Inventors:
• **Fuchs, Carola
82061 Neuried (DE)**
• **Finke, Matthias
82152 Planegg (DE)**

• **Eschrich, Björn
86934 Reichling (DE)**
• **Schmidt, Ronald
82205 Gilching (DE)**

(74) Representative: **Hoffmann Eitle
Patent- und Rechtsanwälte PartmbB
Arabellastraße 30
81925 München (DE)**

(56) References cited:
**WO-A2-2005/114524    WO-A2-2014/144208
US-A1- 2006 237 002    US-A1- 2012 094 600
US-A1- 2015 061 867    US-A1- 2015 174 349**

**Description**

**TECHNICAL FIELD**

The present invention relates to a medical evaluation device, and in particular to a medical evaluation device for evaluating an adherence to a therapy protocol when using an aerosol nebulizer.

**BACKGROUND**

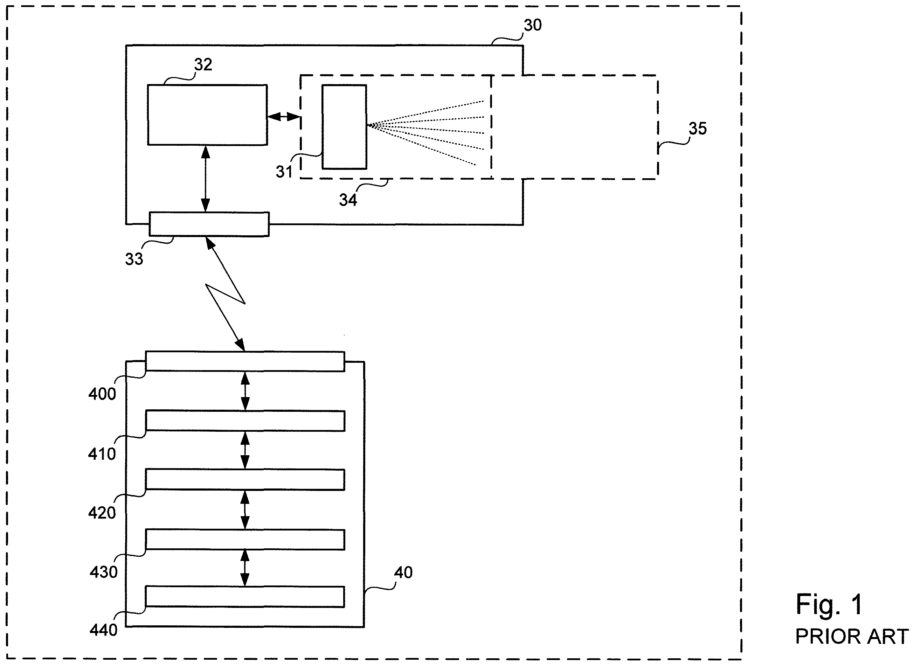

Conventional aerosol nebulizers, such as shown in Fig. 1, are known to have an aerosol nebulizer 30 comprising an aerosol generator 31 to generate an aerosol from a liquid medication. To improve the usability of the aerosol nebulizers for the user, for example for the purpose of a smaller size and light-weight handheld portion of the nebulizer, an external control device 40 may be used. Such a control device 40 may comprise communication interface 400, a processor 410, a memory 420, for example in the form of a volatile and/or nonvolatile memory, a display 430, and an input unit 440 which may be configured in the form of a keyboard, individual buttons, or a touch-sensitive surface on/in the display. Such an external control device 40 may additionally operate to establish a wireless communication connection with the aerosol nebulizer 30 to transfer configuration data to the aerosol nebulizer 30 to appropriately set and control the operation of the aerosol generator 31. Similarly, nebulization data indicating an operation of the aerosol nebulizer and/or indicating a usage of the aerosol nebulizer by the user may be generated and transferred via the wireless communication connection from the aerosol nebulizer 30 to the external control device 40. Such nebulization data, which may include inhalation data and/or measurement data, may be generated, for example, by an internal control unit of the aerosol nebulizer and/or one or more sensors that are mounted at the aerosol nebulizer and/or are connected with the aerosol nebulizer. An example of such an external control device is an external computing device, for example in the form of a smartphone or a PDA as described in DE 102 43 371 A1 or US 2006/0237001 A1. Such an external control device may further exhibit a telecommunication module and may thus further offer the capability to transfer the nebulization data via the internet to a central data base, for example for telemedicine and medical evaluation purposes and for the purpose of (centralized) electronic health records.

However, while attempting to improve the medical success of the inhalation therapy by an improved evaluation process using telemedicine capabilities, users/patients may be very sensitive as to the transfer of personal health care data, including the above nebulization data, to a central server for medical evaluation purposes and to receive a corresponding feedback that may alter a prescribed therapy protocol due to a change of the operation and/or usage of the aerosol nebulizer via an unknown and insecure data channel. As such, confidentiality and integrity of personal health data as well as reliable technical feedbacks are key attributes that are not guaranteed when conventionally transferring data between an aerosol nebulizer and a central server.

Against this background, it is an objective of the present invention to provide a medical evaluation device that overcomes the above technical disadvantages.

US 2006/237002 A2 describes a method of controlling the functioning of a portable medicament dispenser, said portable medicament dispenser being for use with a refill container, said method comprising: (a) providing a memory for storing one or more parameters relating to the functioning of said dispenser; (b) storing authentication data for authenticating data for controlling a function of said dispenser; (c) receiving control data for said dispenser; (d) performing authentication of the control data using said stored authentication data; (e) in dependence on a result of the authentication, activating one or more parameters in said memory to control functioning of said dispenser in accordance with said control data, wherein the step (d) of performing authentication of the control data is performed by said refill container. Further, a set of records are for storing data for transmission to network server, including compliance monitoring data received from a dispenser, and self-test health check data, and self-test health status data records, containing data such as peak flow rate data in the case of a respiratory inhaler or blood glucose level data in the case of an insulin dispenser, which is recorded by a sensor in, or associated with, the docking station.

US 2015/174349 A1 describes devices and methods for monitoring a patient's compliance with an inhaler treatment regimen and assisting in prescription refills. The device may monitor an inhaler's motion to determine whether the motion is characteristic of typical inhaler use. Additionally, the device may monitor a temperature of the inhaler or in proximity to the mouthpiece to determine whether a patient has used the inhaler. The devices and methods may incorporate a smart phone application that provides notifications and alerts to aid in compliance with the medication regimen, as well as facilitating prescription refills.

**SUMMARY**

The features of a medical evaluation device according to the present invention are defined in claim 1. Advantageous embodiments are described in the dependent claims. The features of a computer program according to the present invention are further defined in claim 20. In addition, the features of a system according to the present invention are further defined in claim 21.

## BRIEF DESCRIPTION OF THE DRAWINGS

**Fig. 1** shows a schematic illustration of an aerosol nebulizer control device and an aerosol nebulizer.

**Fig. 2** shows a schematic illustration of a medical evaluation device in communication interaction with a communication device, an aerosol nebulizer control device, and an aerosol nebulizer.

**Fig. 3a** shows an association between an identification value of the control device and an identification value of the communication device.

**Fig. 3b** illustrates an association between an identification value of the control device and an identification value of the communication device in conjunction with a user identification.

**Fig. 4** shows a schematic illustration of a medical evaluation device in communication interaction with a communication device, an aerosol nebulizer control device, and an aerosol nebulizer.

**Fig. 5** shows an example of an inhalation report generated at the medical evaluation device according to an embodiment.

**Fig. 6** shows another example of an inhalation report generated at the medical evaluation device according to an embodiment.

**Fig. 7** shows another example of an inhalation report generated at the medical evaluation device according to an embodiment.

**Fig. 8** shows a schematic illustration of a medical evaluation device in communication interaction with a communication device, an aerosol nebulizer control device, an aerosol nebulizer, and a diagnosis device.

## DESCRIPTION OF THE EMBODIMENTS

Embodiments of the present invention are described with reference to the Figures. It is noted that the following description should not be construed as limiting the invention. In the following and the above, similar or same reference signs indicate similar or same elements or operations.

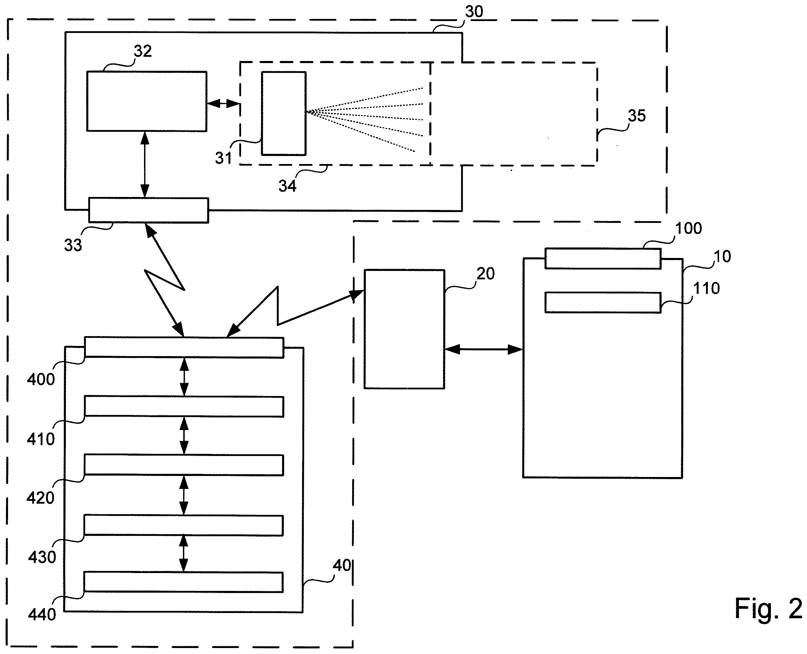

**Fig. 2** shows a schematic illustration of a medical evaluation device 10 according to an embodiment in relation and communication interaction with a communication device 20, an aerosol nebulizer 30, and a control device 40. Here, the medical evaluation device 10 is configured to evaluate an adherence to a therapy protocol when the aerosol nebulizer 30 is used by a user/patient for nebulizing a liquid medication according to the therapy protocol, as will be described below. The evaluation of the adherence to the therapy protocol generally refers to an evaluation as to whether the user/patient performs an inhalation process as prescribed by the therapy protocol and/or as to whether the aerosol nebulizer 30 operates as required by the therapy protocol.

More specifically, the control device 40 according to Fig. 2 is in communication interaction (wireless or wire based, or electronically connected or directly integrated) with the aerosol nebulizer 30 that may comprise an aerosol generator 31, for example a membrane aerosol generator or the like, that is suitable to nebulize a liquid medication, which is held in a reservoir (not shown) of the aerosol nebulizer 30.

The liquid medication may be a fluid that contains at least one medical drug, i.e. an active substance or pharmaceutical ingredient. The liquid medication is transformed into an aerosol by the aerosol generator 31, which subsequently enters an inhalation chamber 34 that is connected to a patient interface 35, e.g. a mouthpiece, face mask, nasal prongs or the like. From the patient interface, the patient/user inhales the aerosol into the respiratory tract to perform a medical treatment of the lungs, throat, nose or sinuses according to the (aerosol) therapy protocol. Further, the aerosol generator 31 operates according to configuration data that may be provided from the control device 40 via a communication interface 33 and an operation unit 32 of the aerosol nebulizer 30. The aerosol nebulizer 30 provides nebulizing data, which may include inhalation data and/or measurement data generated by one or more sensors (not shown) at the aerosol nebulizer 30 and/or connected with the aerosol nebulizer 30, via the communication interaction to the control device 40. Examples of such sensors are sensors for detecting a state of a membrane aerosol generator 31, for detecting a filling level of a liquid medication in a reservoir of the aerosol nebulizer 30, for detecting inhalation/exhalation flow/volume/time characteristics during the use of the aerosol nebulizer 30 of the user/patient, and the like.

As shown in **Fig. 2**, the control device 40 is provided separately from the aerosol nebulizer 30 in order to separate the control functionality as much as possible from aerosol nebulizer 30. This is, however, not considered to be limiting and the control device 40 may alternatively be provided as an integral part of the aerosol nebulizer 30, for example in the form of an integral unit of the aerosol nebulizer, for example as a suitably adapted operation unit 32. In such a case, the communication interface 33 would be configured to establish a wireless communication interaction (as explained below) with the separate communication device 20.

As further shown in **Fig. 2**, the control device 40 may establish a wireless communication connection with the separate communication device 20 and may perform data (configuration data and/or nebulization data) communication with the communication device 20 via the established wireless communication connection. The established wireless communication connection may be a Bluetooth connection, a near field connection, a WiFi connection or the like. Via the established wireless communication connection a data communication between the control device 40 and the separate communication device 20 may be performed so that the control device 40 may send/receive data to/from the external communication device 20. As will be further described below such a data transfer may comprise, for example, the transfer of configuration data for the proper operation of the control device 40 and/or the aerosol generator 31 as well as the transfer of nebulizing data, for example in the form of measurement data and/or inhalation data.

Further, the communication device 20 according to **Fig. 2** may be a customized communication hub, a portable device such as a smartphone, a tablet, or a smart watch or other smart wearable devices having a customized application program, or the like. The communication device 20 may also be a customized communication hub without an input unit and/or a display unit. Further, the communication device 20 may receive an instruction from the medical evaluation device 10 to activate or deactivate the establishment of the wireless communication connection between the nebulizer control device 40 and the communication device 20 (described above). The communication device 20 may thus itself be connected, for example via a cloud server (not shown), with the medical evaluation device 10 to provide a feedback to the control device 40 so that the inhalation therapy and/or the operation of the aerosol nebulizer 30 are suitably adjusted based on an evaluation performed by the evaluation unit 110 (as described below).

According to **Fig. 2**, the medical evaluation device 10 for evaluating the adherence to the therapy protocol comprises a communication unit 100 and an evaluation unit 110. The medical evaluation device 10 may be, for example, a central server or may be a distributed computer network.

The communication unit 100 of the medical evaluation device 10 is configured to establish a communication connection with the communication device 20. This communication connection may be a wireless connection, for example via cloud server, a radio connection using a radio communication module, such as a GSM module, UMTS module, LTE module, with or without a SIM card, or the like. The communication connection may be configured for providing *first* configuration data to the aerosol nebulizer 30, which may be generated by the evaluation unit 110, via the communication device 20 and the nebulizer control device 40. Here, the *first* configuration data are for configuring the aerosol nebulizer 30 according to the established therapy protocol. For example, the first configuration data may include at least one of a current date, a current time, a maximal nebulization duration, a number of maximal storable records, an activation or a deactivation of the automatic data transfer, and an identification of one or more drugs usable for said liquid medication. The first configuration data may also include an identification value of the control device 40, because each data transfer preferably is performed including a check whether the identification values match with a pre-defined association (white list), as described below.

The communication connection between the communication device 20 and the communication unit 100 of the medical evaluation device 10 may be further provided to receive nebulizing data indicating an operation of the aerosol nebulizer 30 from the communication device 20. The nebulizing data may further include inhalation data that indicate a usage of the aerosol nebulizer 30 by the user/patient. For example, the nebulizing data may include at least one of a nebulization date, a nebulization time, a nebulization duration, one or more interruption criteria for switch-off of the aerosol nebulizer, usage status by a user, a fill level of the liquid medication in the reservoir, inhalation/exhalation flow/volume/time characteristics of said user, and a list of one or more drugs used or to be used for said liquid medication. Such nebulizing data may be appropriately generated by the operation unit 32 of the aerosol nebulizer 30, for example by an appropriate processing of acquired sensor signals.

Further, the evaluation unit 110 of the medical evaluation device 10 may be configured to evaluate the adherence of the user and/or the aerosol nebulizer 30 to the predefined therapy protocol. This evaluation may be based on a comparison of said received nebulizing data with respective data/parameters that are defined within the prescribed therapy protocol. For example, as will be described in further detail below, the therapy protocol may define a minimum inhalation time parameter which indicates a time duration which is considered to be required to effectively perform an inhalation with a particular medical drug. Another example of such parameter may be the number of inhalations that should be performed on a single day. Accordingly, the evaluation unit 110 may perform a processing in which the received nebulizing data are assigned to respective (stored) parameters defined for a prescribed (user-specific) therapy protocol, and subsequently a comparison is performed to evaluate whether the received nebulizing data matches with the required parameter(s). If such a matching occurs, e.g. if the user/patient has performed an inhalation process that is sufficiently long as defined by the minimum inhalation time parameter, then this inhalation process is determined to be a valid inhalation process. Otherwise, i.e. if the user/patient has performed an inhalation process that is not sufficiently long as defined by the minimum inhalation time parameter, then this inhalation process is determined to be an invalid inhalation process.

As a result of the comparison described above when evaluating the adherence, the evaluation unit 110 may further generate therapy related feedback data. The generated therapy related feedback data may subsequently be transmitted by the communication unit 100 via the established communication connection to the communication device 20 which transfers the therapy related feedback data to the control device 40, for example via an established wireless communication connection, such as a Bluetooth connection, a near field connection, a WiFi connection or the like.

As will be further described below, the therapy related feedback data may visually and/or audibly indicate at the nebulizer control device 40 that the user/patient has not followed the therapy protocol as prescribed (for example, based on a certain set of parameters that are set for the user) and urges the user/patient to appropriately change the usage of the aerosol nebulizer 30.

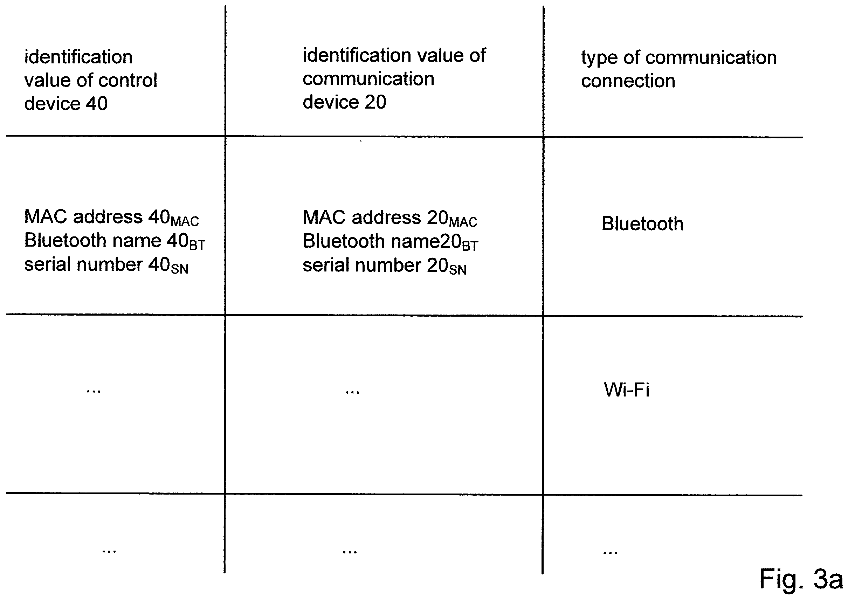

According to a further embodiment, the communication unit 100 may further transmit to the communication device 20 an *association* (also referred to as a white list) between an identification value of the control device 40 and at least one identification value of the communication device 20. This *association* may be generated by the communication unit 100 or a general control unit (not shown) responsive to a corresponding user input at the medical evaluation device 10, and may be stored in a storage unit (not shown) of the medical evaluation device 10, for example in the form of an association table or the like. **Fig. 3a** shows an example of an *association* between an identification value of the control device 40 and an identification value of the communication device 20, here in the form of a MAC address $40_{MAC}$, Bluetooth name $40_{BT}$, and a serial number $40_{SN}$ of the control device 40, and a MAC address $20_{MAC}$, Bluetooth name $20_{BT}$, and a serial number $20_{SN}$ of the communication device 20. Such an association is transferred from the communication unit 100 to the communication device 20. Such a transfer may, for example, occur upon a request from the communication device 20. Such a request may include the identification value of the communication device 20, based on which the medical evaluation device operates to extract a corresponding part from the association table. Based on such a transferred association, the medical evaluation device maintains an external control as to which communication device 20 may be communicatively connected with which control device 40. Upon receiving the *association* from the communication unit 100 of the medical evaluation device 10, the communication device 20 (e.g. Qualcom 2net-Hub, Smartphone and the like) stores the association in an internal storage element (not shown), for example an EEPROM, a memory card, or the like. Further, upon receiving an instruction from the medical evaluation device 10 to activate the establishment of the wireless communication connection between the communication device 20 and the nebulizer control device 40, a control unit (not shown) of the communication device 20 refers to the stored association to determine whether a valid establishment of the wireless communication connection is allowed. As such, the medical evaluation device also maintains an external control as to whether establishment and/or maintenance of the wireless communication connection is activated or deactivated.

As further shown in **Fig. 3a**, the *association* (white list) may further be generated in dependence of the type of wireless communication connection between the control device 40 and the communication device 20. If this wireless communication connection is a Bluetooth connection, for example, then the identification value of the communication device 20 may include at least one of a MAC address, a (Bluetooth friendly) NAME, and a serial number of the communication device 20. Likewise the identification value of the control device 40 may include at least one of a MAC address, a (Bluetooth friendly) NAME, and a serial number of the control device 40, as described above. In addition, appropriate identification values may be defined and stored in the association for a WiFi connection, a near field connection, and the like. The *association* may further be generated and transmitted for a plurality of types of wireless communication connections between the control device 40 and the communication device 20. As a result, different types of wireless communication connections may thus be allowed to be established between the control device 40 and the communication device 20, and the control device may seamlessly switch between a first wireless communication connection, such as Bluetooth, and a second wireless communication connection, such as a near field communication connection.

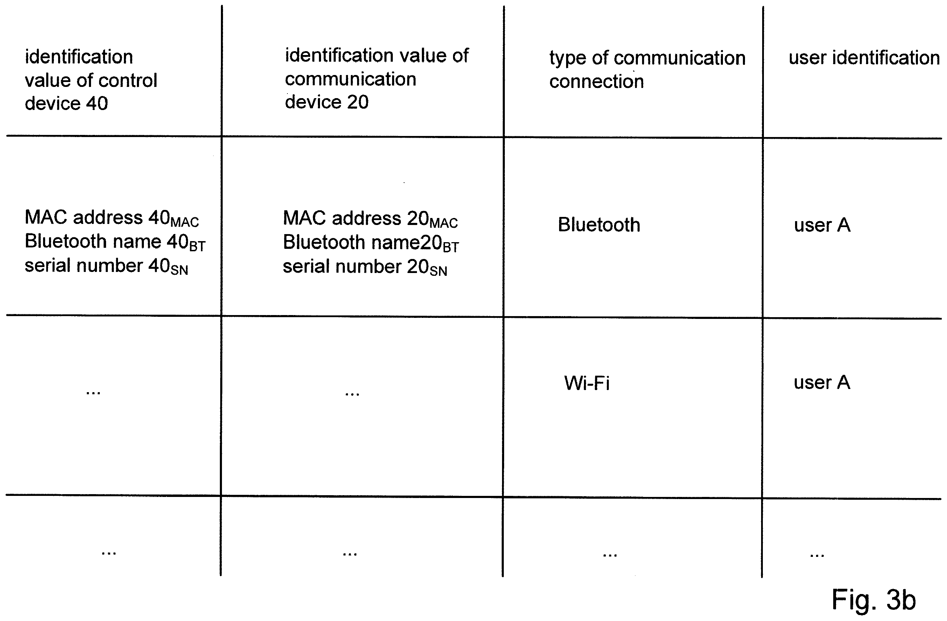

This *association* (white list) defines a *valid* combination of identification values of the control device 40 and the communication device 20 which are thus allowed to establish the wireless communication connection with each other. The establishment of the wireless communication connection between the control device 40 and the communication device 20 is thus allowed/enabled by the transmitted *association.* Importantly, the transmitted association does not include any personal data. Personal data are only linked to the *association* in the medical evaluation device 10. For example, the *association* may be stored in the medical evaluation device 10 in conjunction with a corresponding user identification, for example in a table as shown in **Fig. 3b.** Based on this setting, the medical evaluation device 10 performs a local mapping (matching) of the nebulizing/inhalation/measurement data, which are received together with the association data, to the user/patient ID. While the adherence to the therapy protocol may thus be evaluated in a user-specific way, the data transfer between the medical evaluation device 10 and the nebulizer control device 40 via the communication device 20 does not include any personal data.

According to a further embodiment, the communication unit 100 may be further configured to transmit an instruction to the communication device 20, wherein the instruction is for activating the establishment of the wireless communication connection between the communication device 20 and the nebulizer control device 40. This activation means that establishment of the wireless communication connection may be started based on the transmitted association (white list). Likewise, the communication unit 100 may be configured to transmit an activation or a deactivation instruction to the communication device 20. When receiving such a deactivating instruction, the communication device 20 responds by deactivating and/or disabling the wireless communication connection between the communication device 20 and the nebulizer control device 40, for example by removing the association, setting the association inactive (for example, based on a corresponding flag setting) or the like. Such a deactivation/disabling of the wireless communication connection may be performed when the therapy protocol is finished, when the aerosol nebulizer 30 and/or the communication device 20 has to be replaced, or the like. As such, the medical evaluation device 10 may provide a control function over the wireless communication connection between the communication device 20 and the nebulizer control device 40.

The above *first* configuration data may be data for operating the aerosol nebulizer 30 and/or data for operating the control device 40, and are transferred from the medical evaluation device 10 via the communication connection to the communication device 20 and subsequently via the established wireless communication connection to the control device 40.

In an embodiment, the configuration data may comprise at least one of an identification value of the control device 40, a current date, a current time, a maximum nebulization duration, a number of maximal storable records, an activation or deactivation of the automatic data transfer, and an identification of one or more drugs usable for said liquid medication according to the therapy protocol. The provision of the current time and date may be provided in order to synchronize in time the operation of the control device 40 and/or the operation of the aerosol nebulizer 30 with the medical evaluation device 10 that receives and evaluates nebulization data (inhalation/measurement data). The maximum nebulization duration which may be drug-specific and defines a maximum amount of time that the aerosol generator 30 is allowed/expected to generate aerosol from the liquid medication, and may thus be used by the control device 40 to alert the user/patient to properly clean or replace the aerosol generator or aerosol nebulizer and/or suitably switch-off the aerosol generator 30, and thus to cancel a contradicting command from the user. The user may be informed about such a switch-off due to reaching and/or exceeding the allowed/expected maximum nebulization duration via the control device 40, e.g. via a display 430. Further, the deactivation of the automatic data transfer may indicate to the control device 40 to deactivate inhalation/measurement data transfer to the communication device 20 at the end of an aerosol therapy/inhalation session. Further, the identification of one or more drugs usable for said liquid medication may be notified to the user via the display 430. Further, the number of maximal storable records indicates a maximal number of data records or data sets that are storable in the control device 40. A data record or a data set comprises, for example, all data being related to a single session of the aerosol therapy protocol. Here, the number of maximal storable records is usually fixed, but may be changed according to software and/or hardware updates. The number of maximal storable records may be used in the control device 40 to timely issue a visual and/or audible warning to the user to indicate that a wireless communication connection should be established to transfer the stored data records. Such a warning may be issued when a predetermined number of data records, for example 90 data records of respective sessions of the aerosol therapy protocol have been stored and the number of maximal storable records is 100.

The nebulizing data described above may include at least one of nebulization date, a nebulization time, a nebulization duration, one or more interruption criteria for switch-off of the aerosol nebulizer, a usage status by a user/patient, a fill level of the liquid medication in the reservoir, inhalation/exhalation flow/volume/time characteristics of the user/patient, and a list of one or more drugs used or to be used for said liquid medication.

In particular, the usage status by a user/patient may be detected based on the provision of one or more sensors at and/or in connection with the aerosol nebulizer 30 that are suitably configured to detect whether the user/patient actually performs an inhalation of the aerosol containing the liquid medication. This detection may thus distinguish from a situation in which generated aerosol is simply ejected from the aerosol nebulizer without being inhaled by the user/patient. A detection of such a situation may thus be recorded and communicated to the medical evaluation device as incorrect inhalation. Such a sensor to detect actual inhalation may be a flow sensor, for example a hot wire anemometer, hot film anemometer or a pneumotachograph. In addition, such a detection may be performed by using a microphone or by providing a sensing device at the patient interface 35 of the aerosol nebulizer to detect a contact with the user/patient, and/or by detecting the presence and characteristics of an aerosol (e.g. density) in a mixing chamber of the aerosol nebulizer. Sensing devices and/or flow sensors may be based on different physical techniques, for example optical detection (e.g. by using light, light-emitting diode (LED), laser, photo diode), magnetic detection, acoustical detection (e.g. by using a microphone or a microphone in combination with a loudspeaker), pressure detection (e.g. by using a differential pressure sensor), electrical detection (e.g. by using dynamo-electric effect, or using a piezo-electric element), thermal detection (e.g. by measuring thermal variables), and visual observation (e.g. by a rotameter). All kinds of practicable sensor techniques are applicable whereas a sensor technique is preferred that uses an optical, piezo-electric, acoustic, hot film anemometer and/or pressure detection, which shows a robust and reproducible operation characteristic, e.g. in a flow range from - 100 l/min up to 100 l/min or preferred in a range from - 60 l/min up to 60 l/min and more preferred in a range from - 30 l/min up to 30 l/min in order to detect exhalation and inhalation cycles).

In a further setup other flow ranges may be used, like flow ranges from zero (0 l/min) up to 100 l/min or preferred in a range up to 60 l/min and more preferred in a range up to 30 l/min. In an even further setup other flow ranges may be used, like flow ranges from 5 l/min up to 100 l/min or preferred in a range from 5 l/min up to 60 l/min and more preferred in a range from 5 l/min up to 30 l/min in order to at least detect the inhalation cycle).

The nebulization data may, for example, also be generated by the control device 40 that monitors the operation of the aerosol generator and the aerosol nebulizer 30. Here, the reason for a switch-off may be a manual switch off by the user, a forced switch-off due to reaching the maximum duration for operation (e.g. maximum nebulization duration), absence of a suitable amount of liquid medication, or the like. Here, pre-determined reasons for switch-off may be provided at the control device 40, like e.g. maximum nebulization duration exceeded, no liquid in the fluid reservoir, volume or liquid level in the fluid reservoir at or below a limit, as well as the absence of a user contact or an inhalation maneuver (flow, volume or time characteristics of inhalation/exhalation) of the user. Alternatively, the user may input a reason for switch-off via an operating unit 32 of the control device 40.

For example the usage status by a user of the aerosol nebulizer 30 may be detected by a sensor (not shown) included in the aerosol nebulizer 30. The sensor (not shown) may be included in or attached to the patient interface 35, e.g. a mouthpiece, mixing chamber 34, inhalation/exhalation valves, and/or aerosol generator 31. The sensor sends a signal via the communication to the control device 40 for the purpose of evaluating whether a user/patient actually uses the nebulizer 30. For example, during the aerosol therapy the user/patient is in contact with the patient interface (measured by a contact sensor), inhales through the mixing chamber or the patient interface (e.g. measured by a flow sensor or density sensor), or generates an inhalation flow through the device (e.g. measured by a flow sensor) and thus, one or more corresponding sensor signals are generated, which can be communicated and evaluated at the medical evaluation device 10.

In a further embodiment, the above described reception of nebulizing data at the communication unit 100 of the medical evaluation device 10 may be associated with additionally receiving an identification of the communication device 20 and an identification of the nebulizer control device 40. In other words, the communication device supplements the nebulizing data with data that identify both the communication device 20 and the nebulizer control device 40. The additional *device identification data*, which do not identify any personal data, may be included in a single communication message, for example in the payload thereof. As such, a relevant identification of the nebulization data is done only by the *device identification data*. Responsive to the reception of nebulizing data in conjunction with the identification data, the evaluation unit 110 of the medical evaluation device 10 may refer to the above described association (white list) stored in the storage unit (not shown) of the medical evaluation device 10, to check whether the received identification data are stored as a *valid* combination of identification values of the control device 40 and the communication device 20. In case the received identification data match with a *valid* combination of identification values of the control device 40 and the communication device 20 stored in the storage unit of the medical evaluation device, the evaluation unit 110 proceeds to evaluate the nebulizing data.

The received communication device 20 identification and the received nebulizer control device 40 identification thus define a received identification pair, and only if this received identification pair coincides with an identification pair of the above described association (white list), then the received nebulization data are started to be evaluated in order to determine an adherence to the therapy protocol. Otherwise, the received nebulizing data are ignored and thus may be dropped or otherwise canceled by the evaluating unit 110. In addition, the received nebulizing data may be stored together with the received identification pair in the storage unit (not shown) of the medical evaluation device without a link to personal data. The relevant identification, for example for the purpose of providing the therapy related feedback data, may be performed on the basis of the identification pair of the above described association (white list).

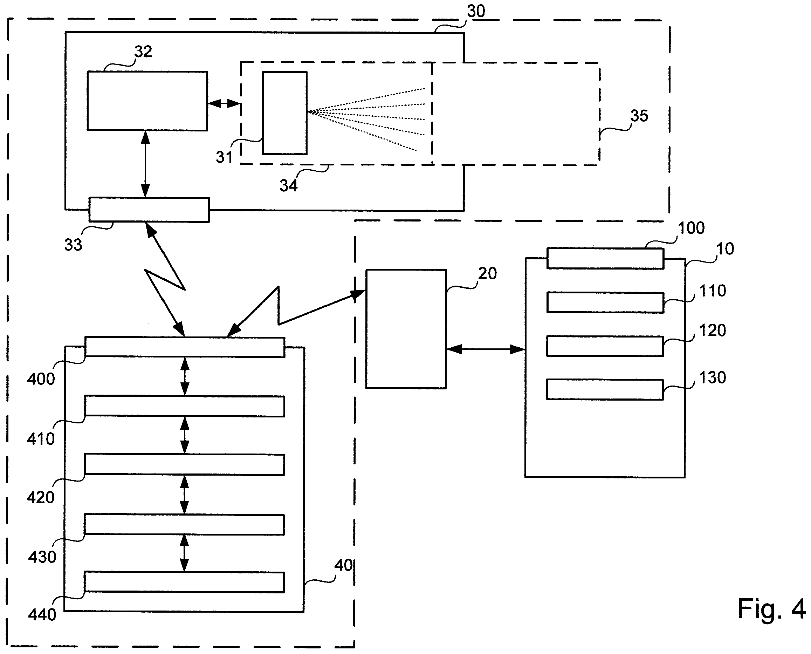

**Fig. 4** shows a medical evaluation device 10 according to a further embodiment. In addition to the medical evaluation device 10 according to Fig. 1, the medical evaluation device 10 further includes an encryption/decryption unit 120. Here, the encryption/decryption unit 120 performs an encryption process on the above described configuration data before a transmission via the established communication connection to the communication device 20 takes place. Likewise, the encryption/decryption unit 120 performs a decryption process on the received nebulizing data which may be encrypted by the nebulizer control device 40. Here, the encryption/decryption processes may be performed by using an encryption/decryption key (e.g. an algorithm, like Advanced Encryption Standard: AES 128 Bit) that is known to the medical evaluation device 10 and the nebulizer control device 40 but remains unknown to the communication device 20.

According to a further embodiment, the evaluation unit 110 may calculate a daily adherence rate and/or a cumulative adherence rate for a defined time period of said therapy protocol (for example, for a plurality of days). Here, the adherence rate may be calculated based on the following expression (1):

$$\text{adherence rate} = \frac{((N_{IV}/N_{IT})_{Day\,1} + (N_{IV}/N_{IT})_{Day\,2} + \ldots + (N_{IV}/N_{IT})_{Day\,n})}{n} \tag{1}$$

in which $N_{IV}$ is a number of valid inhalations, i.e. inhalation sessions, when a predefined required minimum inhalation duration is reached, for example, $N_{IT}$ is a number of inhalations defined according to the prescribed therapy protocol, and n is the number of days in the defined time period. Here, the number of prescribed daily inhalation sessions and the minimum duration of one valid inhalation may be drug specific, user-specific or study-specific (for a clinical trial). Based on the above equation (1), the cumulative adherence rate refers to a ratio between a sum of daily adherence rates of each therapy day within the selected time period of the therapy protocol and the total days of the selected time period. Based on the above equation (1), the daily adherence rate may be determined by considering the adherence rate for n=1.

Here, the nebulizing data received by the medical evaluation device 10 may include respective nebulizing/inhalation start date/times, nebulizing/inhalation end date/times, and nebulizing durations for successive operations of said aerosol nebulizer 30. Based on the received nebulizing data, the evaluation unit 110 may further determine whether a minimum inhalation duration, as prescribed for the therapy protocol, is respectively reached for the successive operations of said aerosol nebulizer 30, i.e. whether a nebulizing/inhalation of the liquid medication is actually performed for the required minimum inhalation duration.

If the evaluation unit 110 determines that the required minimum inhalation duration is reached for an individual nebulizing/inhalation operation of the aerosol nebulizer 30, then this nebulizing/inhalation operation is determined to be valid and the therapy protocol is accordingly adhered to. By contrast, if the required minimum inhalation duration is not reached for an individual nebulizing/inhalation operation of the aerosol nebulizer 30, then this nebulizing/inhalation operation is determined to be non-valid. In addition, if the control device 40 and the aerosol nebulizer 30 are used in connection with a cleaning mode, then data associated with this cleaning mode and indicating success, failure, and/or progress of the cleaning mode are separated from the nebulizing data and are not used for determining the adherence to the prescribed therapy protocol.

The calculated daily adherence rate and/or cumulative adherence rate for a defined time period may be displayed on a display unit 130 of the medical evaluation device 10, as shown in Fig. 2 or 4. For example, the user (may also be clinical trial personnel, a medical doctor, physician or the like) may use a web-interface to access the nebulizing data and the calculated adherence data, and view the data for example in a table view of all inhalation records, graphical view of all inhalation records, and/or an adherence report (to be described later). Such adherence reports may be stored locally in the medical evaluation device or exported to an external device. Such a report may comprise any of a corresponding time period, a patient identification, a study number, and/or a graphical display of daily/cumulative adherence rate over a defined period of said medical protocol.

If the medical evaluation device is used, for example, in clinical trials, then the prescribed therapy protocol (therapy regime) would be defined for a plurality of users/patients, and the medical evaluation device may be used to receive nebulization data from a plurality of aerosol nebulizers, and to evaluate the adherence to the therapy protocol for the plurality of users/patients. If, on the other hand, the medical evaluation device is used to determine adherence for individual users/patients, then a prescribed therapy protocol may be provided for each user/patient individually and the adherence rates may be calculated on an individual user/patient basis. As such, the above reports may be generated for an individual user/patient or a defined user group, e.g. a group of users/patients that participates in a clinical trial.

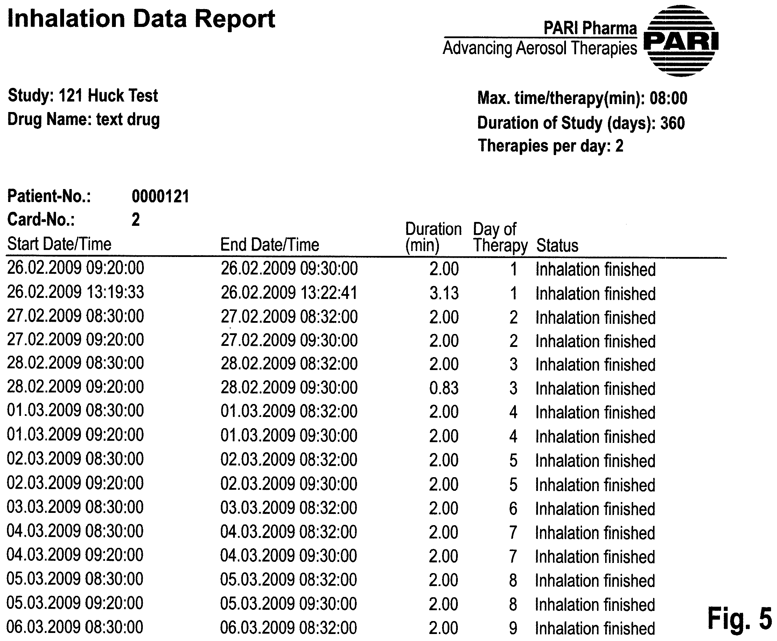

**Figs. 5 - 7** show further examples of the generated adherence reports.

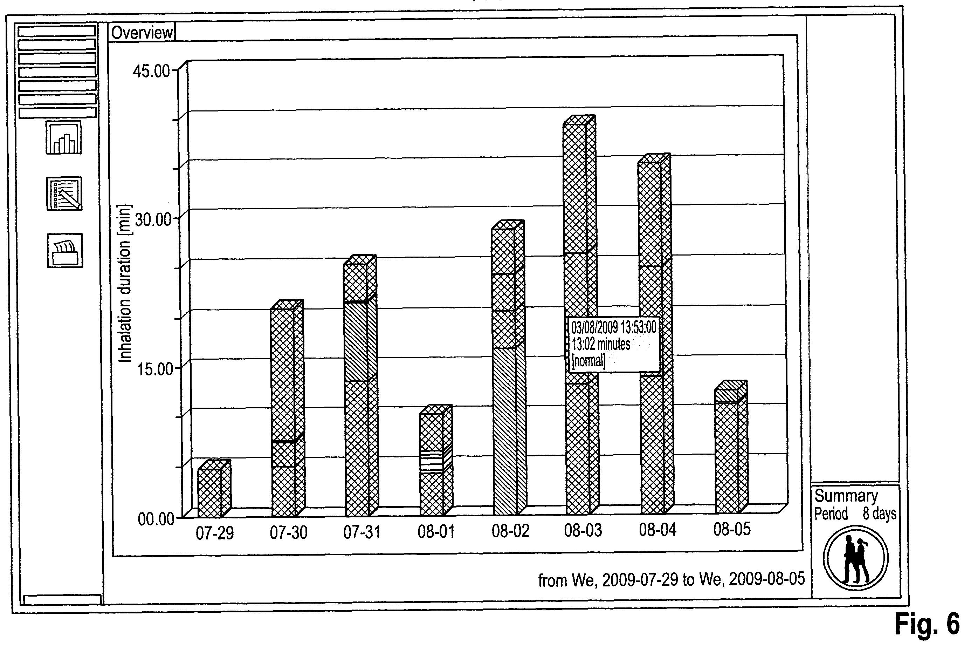

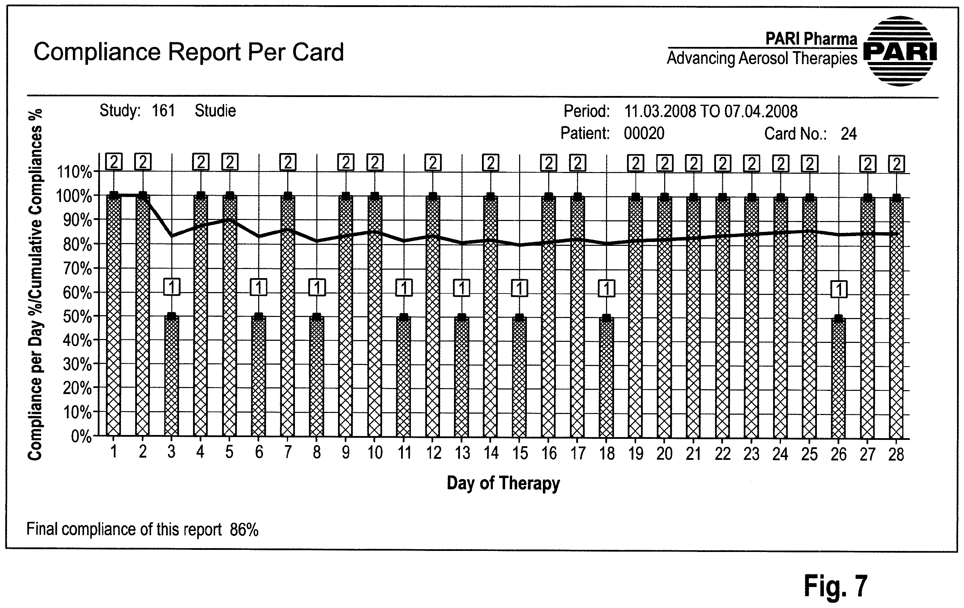

As shown in **Fig. 5**, an individual inhalation report may include respective starting dates/times of the inhalation and corresponding end dates/times, based on which a respective inhalation duration may either be determined in the medical evaluation device or included in the nebulization data that are received by the medical evaluation device. Further, the report may indicate an inhalation day, a status of the inhalation, and a reason for switch off. Further, as shown in the example of **Fig. 6**, an individual inhalation report may graphically display over a horizontal date-axis the respective number of inhalations (or alternatively the date and/or time of inhalation, or the sum of inhalations per day) and duration of each inhalation on the vertical inhalation duration axis. Moreover, the individual inhalations may be distinguished with regard to a plurality of switch-off criteria (for example, via a color code, or the like). Further, as shown in the example of **Fig. 7**, a compliance report may graphically display over a horizontal day of therapy axis an individual compliance per day and a cumulative compliance. As shown, the daily compliance may fluctuate between 0% and above 100%, for example between 50% and 100%, and thus the cumulative compliance may accordingly vary (i.e. increase and decrease). In the example of **Fig. 7**, the cumulative compliance reaches a final compliance at day 28 of 86%. Here, the relevant information that may be included in the report may be at least one of a corresponding time period, a patient number, a study number, a graphical display of a daily/cumulative adherence rate, and an explicit presentation of the calculated cumulated adherence rate for the selected time period. In a further example (not shown), the cumulative compliance may be reported with values above 100%, e.g. 120%, 130%, 150% or even 200%, whereas this deviation will be monitored and/or the patient/nurse/physician/service center may be alerted to interact and prevent overdosing.

In another embodiment the evaluation unit 110 of the medical evaluation device 10 may be further configured to generate the therapy related feedback data, if the daily adherence rate and/or the cumulative adherence rate do not match the respective parameters defined in the therapy protocol. For example, if the measured and calculated daily and/or cumulative adherence rate is below a respective adherence rate that is prescribed for the therapy protocol of the individual user or of a user group (for example in the case of a clinical trial), or if the required minimum inhalation duration is not reached, then corresponding therapy related feedback data are generated by the evaluation unit 110 and subsequently transmitted via the established communication connections to the nebulizer control device 40. The nebulizer control device 40 may be further configured to process the received therapy related feedback data and to thus generate an indication at the nebulizer control device 40. This indication may be in the form of an audible and/or visible signal provided via a display 430 and/or beeper/speaker (not shown) at the nebulizer control device 40 such that the signal indicates to the user that the adherence is not achieved and/or the required minimum nebulization/inhalation duration is not reached. The generated feedback data may also change the configuration of the aerosol nebulizer and/or selectively activate any of the above described sensors in order to generate additional nebulization data for an improved therapy evaluation, or to switch-off any of the above described sensors if particular nebulization data are not required any more.

In a further embodiment, the evaluation unit 110 of the medical evaluation device 10 may be further configured to drug-specifically evaluate the adherence to the prescribed therapy protocol. In particular, the relevant time period over which the therapy protocol is defined, the number of prescribed daily inhalation sessions, and/or the required minimum duration of one valid inhalation may be defined according to the type and/or required volume of the specific drug formulation, i.e. the liquid medication. This enhances the usability and flexibility of the medical evaluation device 10 in providing an appropriate feedback as to the compliance of the user with a drug-specific therapy.

Further the aerosol nebulizers (or inhalation devices) 30 and the evaluation unit 110 may evaluate different respiratory values and/or non-respiratory values, including but not limited to the following values (parameters or variables).

Aerosol nebulizers (or inhalation devices), may be for example nebulizers, jet nebulizers, membrane nebulizers, ultrasonic nebulizers, electronic nebulizers, electronic membrane nebulizers, vibrating membrane nebulizers with a control device 40 (controller), like the eFlow from PARI with a nebulizer control device 40 (e.g. eBase® or eTrack® control device 40), as well as dry powder inhalers (DPIs), metered dose inhalers (MDIs). The medical evaluation device 10 may be, for example, such an aerosol nebulizer and at least one sensor in form of an add-on device and/or included in the controller (i.e. nebulizer control device 40) and/or in the aerosol nebulizer.

The non-respiratory values (parameters or variables) may comprise, but are not limited to the following: type (of study), header (e.g. 6 characters/letters), study-name, patient-number, study-duration, day-of-therapy, status, report-start, report-end, inhalation-date-start, inhalation-date-end, drug-ID, therapies-per-day, drug-name, extension-study-ID, device-serial-number, Bluetooth-network-ID, current-local-time, current-local-date, activation-time, deactivation-time, minimal-therapy-duration, minimum-inhalation-duration, maximal-therapy-duration, maximum-nebulization-duration, and so on.

The respiratory values may comprise, but are not limited to the following: inhalation-date, inhalation-time, inhalation duration, inhalation-flow, inhalation-volume, minimal-therapy-duration, minimum-inhalation-duration, maximal-therapy-duration, maximum-nebulization-duration, spirometric values. All values (respiratory and non-respiratory values) may be expressed as nominal values, target values, nominal ranges, target ranges and so on. In addition, based on the provided sensor data, a tidal volume, inhalation/exhalation ratio, inhalation pauses and other inhalation characteristics may be taken into account by the evaluation unit 110.

Based on the evaluation, the therapy related feedback data may also include a change of configuration parameters, such as the operating frequency, power of the aerosol generator, or with regard to sensor operating parameters, such as target values for a fluid-presence sensor.

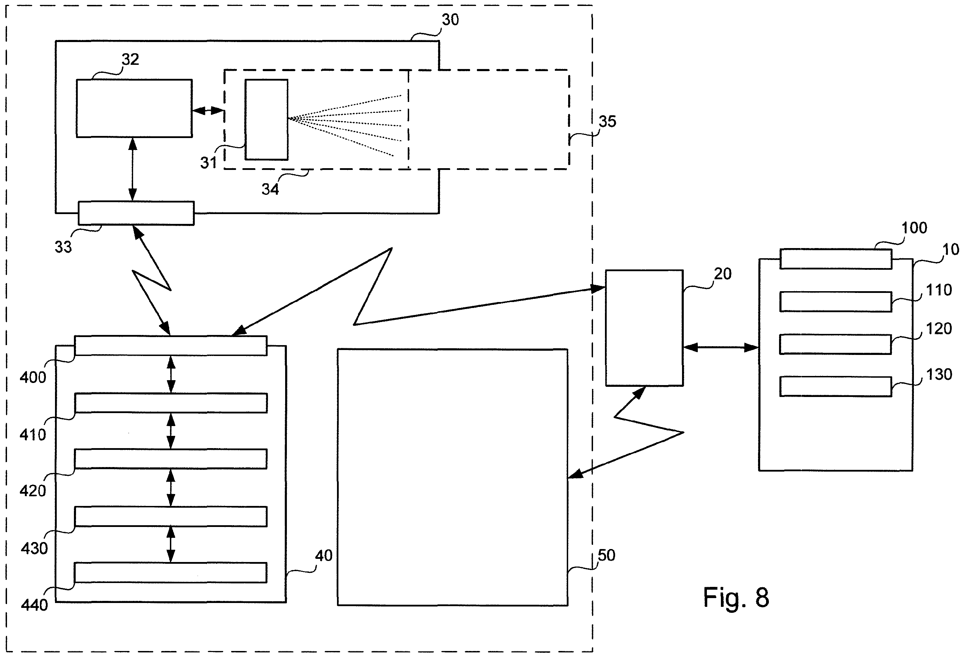

**Fig. 8** shows another embodiment of the medical evaluation device. In comparison to the embodiments illustrated in **Fig. 2 or Fig. 4**, the medical evaluation device further communicates (via the communication device 20) with and controls an additional diagnosis device 50. This diagnosis device 50 may be a device that monitors a state of the respiratory tract and/or a state and/or function of the lung of the user/patient. An example of such a diagnosis device 50 is a spirometer that is able to measure the air volume inhaled/exhaled by the lungs of the user/patient. Further, a spirometer may measure a ventilation, i.e. the movement of air into and out of the lungs. As known, there are various types of spirometers which use a number of different methods for measurement, for example pressure transducers, ultrasonic transmitters. Alternatively or additionally, the diagnosis device 50 may be a device for acquiring non-respiratory parameters (i.e. systemic parameters).

Here, the medical evaluation device 10 may further be adapted to evaluate the adherence to the prescribed therapy protocol when additionally using the diagnosis device 50 to monitor the state of the respiratory tract and/or the state and/or function of the lung of the patient. Here, the diagnosis device 50 may also be used according to the prescribed therapy protocol. For example, the therapy protocol may indicate a point in time, at which a diagnosis using the diagnosis device 50 should be performed. Examples of such a prescribed setting may be specific dates and/or times during the time period defined in the therapy protocol, performing the diagnosis respectively after a defined number of inhalations, such as after every 5 inhalations, or the like.

Further, the medical evaluation device 10 may be configured to generate *second* configuration data for the diagnosis device 50. The *second* configuration data are data to appropriately set and control the operation of the diagnosis device 50. The operation of the diagnosis device 50 may further be set by the *second* configuration data according to the prescribed therapy protocol, as described above.

For example, the *second* configuration data may comprise at least one of respiratory values and additional non-respiratory values (e.g. systemic values, like heart rate, blood pressure, oxygen saturation and so on). In particular, the respiratory values may include at least one of respiratory data, especially respiratory frequency, an exhalation or inhalation flow, or an inhalation volume. Moreover, the *second* configuration data may comprise threshold values with regard to a visual/optical feedback indicator as to the proper usage ("usage support") of the diagnosis device (such as a spirometer) and/or with regard to proper diagnosis data. For example, a red/yellow/green feedback indication at the diagnosis device may be provided. The respective threshold values (nominal values, target values) may be appropriately set by the medical evaluation device in dependence of the therapy protocol, for example in dependence of personal data which are (only) provided/stored at the medical evaluation device and are not transferred according to the concept of the present invention. Such (user-specific) nominal values may indicate a number of diagnosis measurements within a prescribed time period (for example, in order to perform one spirometry measurement per week, otherwise a reminder message or warning message is triggered by the evaluation unit). Further, the setting of appropriate threshold values and/or the selection of suitable diagnosis data (for example, with regard to a specific series of diagnosis devices, such as spirometer of series A, B, C or the like) may be performed automatically, for example based on corresponding settings as defined in a database or are provided by a physician. As such, the evaluation unit 110 also generates diagnosis-related feedback data which are transferred to the diagnosis device 50.

The communication unit 100 of the medical evaluation device 10 may send the generated second configuration data via the communication device 20 to the diagnosis device 50, by using the communication connection established between the medical evaluation device 10 and the communication device 20 (as described above) and by using a second wireless communication connection between the communication device 20 and a control device (not shown) for the diagnosis device 50. This second communication connection is established in the same manner as the above described wireless communication connection between the communication device 20 and the control device 40 for the aerosol nebulizer 30. Further, the communication unit 100 of the medical evaluation device 10 may receive diagnosis data (respiratory values and/or non-respiratory values) that are acquired by the diagnosis device 50 via the communication connection established between the medical evaluation device 10 and the communication device 20. Here, the diagnosis data may indicate the state of the respiratory tract and/or the state or function of the lung of the patient. In addition, or alternatively, the diagnosis data may indicate the heart rate, blood pressure and/or oxygen saturation of the user/patient, for example during the inhalation of the nebulized liquid medication or at a specific time point after the inhalation of the nebulized liquid medication. Moreover, the diagnosis data may include spirometry values with regard to at least one of, for example: Vital Capacity (VC), Forced Vital Capacity (FVC), Total Lung Capacity (TLC), Total Capacity (TC), Inspiratory Vital Capacity (FVC), Tidal Volume (TV, or AZV), Forced Expiratory Volume in one second (FEV1), Relative Capacity of one second (FEV1/FVC), Maximal Expiratory Flow by 50% FVC (FEF50), Maximal Expiratory Flow by 75% FVC (FEF75), Maximal Expiratory Pressure (MEP), Peakflow (PEF), "Z-Score" = (y - Y)/RSD, i.e. the difference between observed (y) and predicted (Y) value divided by the residual standard deviation (RSD) about the mean predicted value used as evaluation assistant, history of measurements or change of measurements as rating or evaluation, for example as an indicator for worsening or bettering of the disease as well as intelligent prediction as to the course of disease and/or patient compliance/acceptance (in the best case prediction of exacerbations), blood values, such as i.e. oxygen saturation ($O_2$), pulse, temperature, inflammatory values/markers (i.e.: White blood cells (WBCs), also called leukocytes or leucocytes, C-reactive protein - CRP, al-Acid glycoprotein, haptoglobin, coeruloplasmin and fibrinogen, as well as interleukins - IL, i.e. IL1, up to IL31, i.e. IL6 for liver as well as IL4, IL5, IL9 and IL13 for asthma and their anti-blockers for a treatment procedure), cough monitor values (i.e. cough frequency), and/or exhalation values (for example, exacerbations counter/marker, and/or gas values, like oxygen - $O_2$, Fractionized Exhaled Nitrogen Oxide - "FENO", Nitric Oxides - "$NO_x$", Carbon Dioxide - "$CO_2$", , Interleukins - "IL" see above, and/or inert gas fractions (i.e. helium [He], neon [Ne], argon [Ar], krypton [Kr], xenon [Xe], and the radioactive radon [Rn]).

More values and parameter, like FEV1, inflammatory values/markers ranges (value < 26 ppb [parts per billion] = healthy), are defined i.e. by the American Thorax Society (ATS) and European Respiratory Society (ERS).

One or a combination of the above values may be used by the evaluation unit 110 for a predicted parameter range and, based on the therapy related feedback data, this may be shown as an optical or acoustic signal to the user or patient. For example, a combination of a green, yellow, and red signal light may be used to show the graduation of a correct inhalation (flow, time and/or volume), like green - optimal inhalation conditions, yellow -limited inhalation conditions (not optimal, yet acceptable), red - unacceptable inhalation conditions.

Due to the therapy related feedback data extensive information can be provided also on personal best values of measurements, applications, inhalations and/or nebulizer device and diagnosis device uses, which may be differentiated with respect to home uses, uses in the physician's office, uses in the hospital, uses of one or a plurality of nebulizer/diagnosis devices. This may be achieved by providing the nebulizer device and/or diagnosis device with a tracking function to monitor the location as to where the nebulization/diagnosis is performed.

For fluid presence sensors used with membrane nebulizers special values and parameters may be used, transferred and adapted, like threshold values, frequency and power consumption of the membrane during operation, each with respect to one or more different drug formulations as well as with respect to membrane material and/or design changes and/or production process changes.

In addition, as input for the medical evaluation device 10, the eating behavior, movement behavior, and/or sport/exercise behavior of the user/patient may be tracked or measured, for example via a smartphone, tablet, glasses (e.g. google glasses), a smart watch, wristband devices (e.g. Google, Apple, Garmin) or other smart wearable devices.

Further, the medical evaluation device 10 may use or access one or a plurality of data bases, for example with regard to reference values for private use by different user or patient groups. These data bases (e.g. physician, hospital and anonymous databanks, like IBM Watson or Apple Health) may include for example information data (values or parameters) relating to a disease, age group, gender, ethnic group and the like.

Further, the medical evaluation device 10 may use the established communication and data transfer capability to implement a reminder function and/or update function and/or a calendar function at the control device 40 and/or the diagnosis device 50. This may be used for the user or patient as a reminder or update for the application, therapy, measurement and/or diagnosis (e.g. therapy protocol, i.e. number and time or detailed schedule of inhalations per day, intervals for spirometer measurements and so on), the need for replacement of components (e.g. change of aerosol nebulizer, change of aerosol generator or other replacement items), cleaning and/or disinfection intervals, need for service (e.g. drift of sensor calibration values, e.g. sensor variance greater than +/-3%), service contact information, optional features, software updates, new software features, updates of instructions for use, and/or environmental conditions, like temperature, humidity, and/or pressure as well as therapy plan coordinated with the physician. These reminder functions and/or update functions and/or calendar functions may be shown optically or acoustically, e.g. on a display, via light indicators (e.g. LEDs), a buzzer, a speaker, but also on an external smartphone, tablet, laptop and/or computer (e.g. via an App, a program, web portal, push notes, E-Mails, SMS, MMS, WhatsApp and so on).

All values, parameters and ranges may be adapted via the medical evaluation device 10 using a communication unit 100, configured to establish a communication connection with a communication device 20. These changes may be shown optically or acoustically, e.g. on a display, via light indicators (e.g. LEDs), a buzzer, a speaker, a smartphone, tablet, laptop and/or computer. These "intelligent" devices may also be used for animation ("gamification") of the application, therapy, measurement and/or diagnosis of the medical evaluation device 10.

Based on the above, the evaluation unit 110 may further use the received diagnosis data for adapting the therapy protocol. Based on the additional provision and usage of the received diagnosis data, the effectiveness of the inhalation therapy may be judged and potentially corrected in real-time. Further, adverse reactions of the user/patients to the prescribed inhalation therapy or the effectiveness/ineffectiveness of the prescribed inhalation therapy may be determined in real-time, and the therapy protocol may be suitably corrected. Such a correction of the therapy protocol may be achieved by the provision of the above-described therapy-related feedback data and/or by transmitting corrected first configuration data to the control device 40. Here, the corrected first configuration data are configuration data that are adapted due to the concurrent diagnosis. For example, based on the concurrent evaluation of diagnosis data and nebulizing data, first configuration data may be generated and transmitted to the nebulizer control device 40 to remind the patient via a display 430 to perform his treatment or diagnosis and which may instruct the aerosol nebulizer to inhibit or interrupt the generation of aerosol until the user/patient has performed a diagnosis of the state of the respiratory tract and/or the state of the lung of the patient.

In addition, personal best values may be determined by the evaluation unit 110, both with regard to an evaluation of the nebulizing data and with regard to an evaluation of the diagnosis data. Such personal best values may be determined with regard to whether the usage of the aerosol nebulizer and/or the diagnosis device occurs at home or in the physician's office. Preferably, the usage of the aerosol nebulizer and/or the diagnosis device at home or in the physician's office may be determined based on the defined association (white list) which defines a unique combination of the aerosol nebulizer and diagnosis devices with a specific communication device (which identifies the location where the devices are used).

The feedback data (therapy related and/or diagnosis related) may further include service data with regard to the device operation. For example, the service data may include an indication as to a validity and/or usability of a sensor (which is thus requested to be updated). In addition, the feedback data may trigger a predictive maintenance operation at the diagnosis device or the aerosol nebulizer. For example, such a predictive maintenance operation may determine a current power consumption of the respective device. In addition, the feedback data may trigger a timely cleaning of the respective device (e.g. visual feedback via display, potentially in combination with blocking a further aerosol generation and/or further operation of the diagnosis device, etc.). In addition, the feedback data may trigger a self-calibration of the above-described sensors, based on which the evaluation unit 110 determines whether sensor deviations are above an allowed limit or not. If such an evaluation indicates that the deviations are too large, then a further feedback message may request the user to notify/confirm a service. In addition, the feedback data from the medical evaluation device 10 may prompt push messages or the like to indicate functionality updates (firmware update or the like) to the control device 40, aerosol nebulizer 30, and/or diagnosis device 50. Based on such push messages, the respective device(s) may be triggered to directly perform the update (including a status feedback to the medical evaluation device 10 indicating success/failure of the service update). For example, such service updates may include new configuration parameters, for example, to accommodate a new/modified version of an aerosol generator, or new sensor operating parameters, such as new/modified target values for a fluid-presence sensor.

In addition, an application software ("mobile app"), running on a mobile device, may be provided with access to the medical evaluation device 10, and may retrieve corresponding data, evaluation results and the like that have been transferred between the medical evaluation device 10 and the diagnosis device 50, the nebulizer control device 40, and the aerosol nebulizer 30. Such an application software may also be integrated into a larger health care related application software, such as HealthKit by Apple or the like.

The following provides an additional example of personal data as well as configuration data, for example with regard to a spirometer, which are provided at the medical evaluation device 10:

Personal data:

| • Name: | Mustermann, Manfred |
|---|---|
| • Gender: | male |
| • Date of Birth/Age: | 01.08.1978 / 37 years |
| • Size: | 183 cm |
| • Patient-ID: | A123f4 |
| • Weight: | 72 kg |
| • Note | Patient needs intensive support for performing correct inhalation maneuver. |
| • Assigned device(s): | mySpiroSense 719 |

Configuration data:

| • Device ID: | 719 |
|---|---|
| • Measurement unit ID: | 585 |
| • Device type: | mySpiroSense (spirometer) |
| • Firmware Version: | 1.0.17 |
| • Hardware Version: | 1.0.2 |
| • Last calibration: | 09.10.2015 |
| • Calibration valid: | 08.10.2016 |
| • (Reference-) Mode: | FEV1 |
| • Type 1: | Custom (i.e. set by physician (otherwise personal best, reference value) |
| • Target value 1: | 4,59 L |
| • Number of measurements: | 35 |

According to another embodiment, a computer program is provided that includes instructions which, when executed on one or a plurality of data processors, cause the data processor or the plurality of data processors to implement the medical evaluation device 10 as described above.

The above respective modules/units of the medical evaluation device 10 may thus be implemented by a respective processing unit (CPU) that includes one or a plurality of processors, a microprocessor or other processing logic that interprets and executes instructions as defined by the computer program and stored in a main memory. The main memory may include a RAM or other type of dynamic storage device that may store information and instructions for execution by the respective modules/units. For example, the evaluation unit and/or the encryption/decryption unit discussed above may be realized by the processing unit. The ROM may include a ROM device or another type of static storage device that may store static information and instructions for use by the processing unit.

The medical evaluation device 10 may perform these operations in response to the processing unit executing software algorithms contained in a computer-readable medium, such as the main memory, ROM and/or storage device. A computer-readable medium may be defined as a physical or a logical memory device. For example, a logical memory device may include memories within a single physical memory device or distributed across multiple physical memory devices. Each of the main memory, ROM and storage device may include computer-readable media with instructions as program code. The software algorithms may be read into the main memory for another computer-readable medium, such as a storage device or from another device via the communication interface.

The software algorithms contained in the main memory may cause the processing unit(s) including a data processor, when executed on the processing unit, to perform operations or processes described herein. Alternatively, hard-wired circuitry may be used in place of or in combination with the software algorithms to implement processes and/or operations described herein. Thus, implementations described herein are not limited to any specific combination of hardware and software.

Further, the respective units of the medical evaluation device may be implemented in hardware, software, Field Programmable Gate Arrays (FPGAs), application-specific integrated circuits (ASICs), firmware or the like.

It will be apparent to those skilled in the art that various modifications and variations can be made in the entities and methods of this invention as well as in the construction of this invention without departing from the scope of the invention.

The invention has been described in relation to particular embodiments and examples which are intended in all aspects to be illustrative rather than restrictive. Those skilled in the art will appreciate that many different combinations of hardware, software and/or firmware will be suitable for practicing the present invention.

## Claims

1. Medical evaluation device (10) for evaluating an adherence to a therapy protocol when using an aerosol nebulizer (30) having an aerosol generator (31) for nebulizing a liquid medication according to said therapy protocol, said medical evaluation device (10) comprising:

   a communication unit (100), configured to establish a communication connection with a communication device (20), said communication connection being configured for:

   - providing first configuration data to a nebulizer control device (40) of said aerosol nebulizer via said communication device (20), said first configuration data for configuring said aerosol nebulizer according to said therapy protocol;
   - receiving nebulizing data indicating an operation of said aerosol nebulizer from said communication device (20) ;

   an evaluation unit (110), configured to evaluate said adherence to said therapy protocol based on a comparison of said received nebulizing data with respective parameters defined within said therapy protocol; wherein said evaluation unit (110) is further configured to generate therapy related feedback data, wherein said medical evaluation device (10) is further adapted for evaluating the adherence to said therapy protocol when additionally using a diagnosis device (50) for monitoring a state of the respiratory tract and/or a state and/or function of the lung of the patient, wherein said communication connection is further established for:

   - providing second configuration data for a diagnosis control device of said diagnosis device (50) via said communication device (20), said second configuration data for configuring said diagnosis device (50) according to said therapy protocol; and
   - receiving diagnosis data indicating said state of the respiratory tract and/or said state and/or function of the lung of the patient,

   wherein said medical evaluation device (10) is further configured to use said diagnosis data when adapting said therapy protocol.

2. Medical evaluation device (10) according to claim 1, wherein said communication unit (100) is further configured to transmit an association between an identification value of the control device (40) and an identification value of the communication device (20) to the communication device (20).

3. Medical evaluation device (10) according to one of claims 1 - 2, wherein said communication unit (100) is further configured to transmit an instruction to said communication device (20) to activate establishment of a wireless communication connection between said communication device (20) and said nebulizer control device (40).

4. Medical evaluation device (10) according to claim 3, wherein said communication unit (100) is further configured to transmit a deactivation instruction to said communication device (20) to deactivate said wireless communication connection between said communication device (20) and said nebulizer control device (40).

5. Medical evaluation device (10) according to one of claims 1 - 4, wherein the first configuration data comprise at least one of an identification value of said control device (40), a current date, a current time, a maximum nebulization duration, a number of maximal storable records, a deactivation of the automatic data transfer, and an identification of one or more drugs usable for said liquid medication.

6. Medical evaluation device according to one of claims 1 - 5, wherein the nebulizing data comprise at least one of a nebulization date, a nebulization time, a nebulization duration, one or more interruption criteria for switch-off of the aerosol nebulizer, usage status by a user, a fill level of the liquid medication in the reservoir, inhalation/exhalation flow/volume/time characteristics of said user, and a list of one or more drugs used for said liquid medication.

7. Medical evaluation device (10) according to one of claims 1 - 6, wherein said receiving of said nebulizing data is associated with receiving an identification of said communication device (20) and an identification of said nebulizer control device (40).

8. Medical evaluation device (10) according to one of claims 1 - 7, wherein said evaluation unit (110) is further configured to evaluate said adherence only if said received identification of the communication device (20) and said received identification of the nebulizer control device (40) coincide with a stored identification pair.

9. Medical evaluation device (10) according to one of claims 1 - 8, further comprising an encryption/decryption unit (120), said encryption/decryption unit (120) being configured for encrypting said configuration data and for decrypting said nebulizing data.

10. Medical evaluation device (10) according to one of claims 1 - 9, wherein said evaluation unit (110) is further configured to calculate a daily adherence rate and/or a cumulative adherence rate for a defined time period of said therapy protocol.

11. Medical evaluation device (10) according to one of claims 1 - 10, wherein said evaluation unit (110) is further configured to generate an adherence report for each user or a defined user group.

12. Medical evaluation device (10) according to one of claims 1 - 11, wherein said nebulizing data include respective nebulizing start times and nebulization durations for successive operations of said aerosol nebulizer (30), and wherein said evaluation unit (110) is further configured to determine whether a required minimum inhalation duration is respectively reached for the successive operations of said aerosol nebulizer (30).

13. Medical evaluation device (10) according to one of claims 1 - 12, wherein said evaluation unit (110) is further configured to generate said therapy related feedback data if said daily adherence rate and/or said cumulative adherence rate do not match the respective parameters defined in the therapy protocol.

14. Medical evaluation device (10) according to one of claims 1 - 13, wherein said therapy related feedback data is configured to generate an indication at the nebulizer control device (40).

15. Medical evaluation device (10) according to one of claims 1 - 14, wherein said evaluation unit (110) is further configured to drug-specifically evaluate said adherence to said therapy protocol.

16. Medical evaluation device (10) according to one of claims 1 - 15, wherein said diagnosis device (50) is a spirometry device and/or a device for measuring non-respiratory parameters.

17. Medical evaluation device (10) according to claim 1, wherein the second configuration data comprise at least one of respiratory values and non-respiratory values.

18. Medical evaluation device (10) according to claim 17, wherein the respiratory values include respiratory data, especially respiratory frequency, an exhalation or inhalation flow, inhalation/exhalation duration and/or an inhalation volume.

19. Medical evaluation device (10) according to one of claims 1, 17, or 18, wherein said medical evaluation device (10) is further configured to use said diagnosis data when adapting said first configuration data for said nebulizer control device.

20. Computer program including instructions configured, when executed on one or a plurality of data processors interacting via a communication device with a nebulizer control device, an aerosol nebulizer and a diagnosis device, to cause the data processor or the plurality of data processors to implement a medical evaluation device (10) according to any one of claims 1 - 19.

21. System, comprising:

a medical evaluation device (10) according to any one of claims 1 - 19;
a communication device (20);
a nebulizer control device (40); and
an aerosol nebulizer (30), further comprising said diagnosis device (50).

**Patentansprüche**

1. Medizinische Auswertevorrichtung (10) zum Auswerten einer Einhaltung eines Therapieprotokolls bei Verwendung eines Aerosolverneblers (30) aufweisend einen Aerosolgenerator (31) zum Vernebeln eines flüssigen Medikaments gemäß dem Therapieprotokoll, wobei die medizinische Auswertevorrichtung (10) umfasst:

eine Kommunikationseinheit (100), die konfiguriert ist, um eine Kommunikationsverbindung mit einer Kommunikationsvorrichtung (20) aufzubauen, wobei die Kommunikationsverbindung konfiguriert ist zum:

- Bereitstellen erster Konfigurationsdaten an eine Verneblersteuervorrichtung (40) des Aerosolverneblers über die Kommunikationsvorrichtung (20), wobei die ersten Konfigurationsdaten zum Konfigurieren des Aerosolverneblers gemäß dem Therapieprotokoll sind;
- Empfangen von Verneblungsdaten, die einen Betrieb des Aerosolverneblers angeben, von der Kommunikationsvorrichtung (20);

eine Auswerteeinheit (110), die konfiguriert ist, um die Einhaltung des Therapieprotokolls basierend auf einem Vergleich der empfangenen Verneblungsdaten mit entsprechenden, innerhalb des Therapieprotokolls definierten Parametern auszuwerten;
wobei die Auswerteeinheit (110) ferner konfiguriert ist, um therapiebezogene Rückmeldedaten zu erzeugen, wobei
die medizinische Auswertevorrichtung (10) ferner zum Auswerten der Einhaltung des Therapieprotokolls ausgelegt ist, wenn sie zusätzlich eine Diagnosevorrichtung (50) zum Überwachen eines Zustands der Atemwege und/oder eines Zustands und/oder einer Funktion der Lunge des Patienten verwendet,
wobei die Kommunikationsverbindung ferner aufgebaut ist zum:

- Bereitstellen von zweiten Konfigurationsdaten für eine Diagnosesteuervorrichtung der Diagnosevorrichtung (50) über die Kommunikationsvorrichtung (20), wobei die zweiten Konfigurationsdaten zum Konfigurieren der Diagnosevorrichtung (50) gemäß dem Therapieprotokoll sind; und
- Empfangen von Diagnosedaten, die den Zustand der Atemwege und/oder den Zustand und/oder die Funktion der Lunge des Patienten angeben,

wobei die medizinische Auswertevorrichtung (10) ferner konfiguriert ist, um die Diagnosedaten beim Anpassen des Therapieprotokolls zu verwenden.

2. Medizinische Auswertevorrichtung (10) nach Anspruch 1,
wobei die Kommunikationseinheit (100) ferner konfiguriert ist, um eine Zuordnung zwischen einem Identifikationswert der Steuervorrichtung (40) und einem Identifikationswert der Kommunikationsvorrichtung (20) an die Kommunikationsvorrichtung (20) zu übertragen.

3. Medizinische Auswertevorrichtung (10) nach einem der Ansprüche 1 bis 2,
wobei die Kommunikationseinheit (100) ferner konfiguriert ist, um eine Anweisung an die Kommunikationsvorrichtung (20) zu übertragen, um den Aufbau einer drahtlosen Kommunikationsverbindung zwischen der Kommunikationsvorrichtung (20) und der Verneblersteuervorrichtung (40) zu aktivieren.

4. Medizinische Auswertevorrichtung (10) nach Anspruch 3,
wobei die Kommunikationseinheit (100) ferner konfiguriert ist, um einen Deaktivierungsbefehl an die Kommunikationsvorrichtung (20) zu übertragen, um die drahtlose Kommunikationsverbindung zwischen der Kommunikationsvorrichtung (20) und der Verneblersteuervorrichtung (40) zu deaktivieren.

5. Medizinische Auswertevorrichtung (10) nach einem der Ansprüche 1 bis 4, wobei die ersten Konfigurationsdaten mindestens einen von einem Identifikationswert der Steuervorrichtung (40), ein aktuelles Datum, eine aktuelle Uhrzeit, eine maximale Verneblungsdauer, eine Anzahl von maximal speicherbaren Datensätzen, eine Deaktivierung der automatischen Datenübertragung und eine Identifizierung eines oder mehrerer für das flüssige Medikament verwendbarer Medikamente umfassen.

6. Medizinische Auswertevorrichtung nach einem der Ansprüche 1 bis 5, wobei die Verneblungsdaten mindestens eines von einem Verneblungsdatum, einer Verneblungszeit, einer Verneblungsdauer, einem oder mehreren Unterbrechungskriterien zum Abschalten des Aerosolverneblers, einem Verwendungsstatus durch einen Benutzer, einem Füllstand des flüssigen Medikaments im Behälter, Inhalations-/Exhalationsstrom-/Volumen-/Zeitmerkmalen des Benutzers und einer Liste eines oder mehrerer für das flüssige Medikament verwendeter Medikamente umfassen.

7. Medizinische Auswertevorrichtung (10) nach einem der Ansprüche 1 bis 6, wobei das Empfangen der Verneblungsdaten dem Empfangen einer Identifikation der Kommunikationsvorrichtung (20) und einer Identifikation der Verneblersteuervorrichtung (40) zugeordnet ist.

8. Medizinische Auswertevorrichtung (10) nach einem der Ansprüche 1 bis 7, wobei die Auswerteeinheit (110) ferner konfiguriert ist, um die Einhaltung nur dann zu bewerten, wenn die empfangene Identifikation der Kommunikationsvorrichtung (20) und die empfangene Identifikation der Verneblersteuervorrichtung (40) mit einem gespeicherten Identifikationspaar übereinstimmen.

9. Medizinische Auswertevorrichtung (10) nach einem der Ansprüche 1 bis 8, die ferner eine Verschlüsselungs-/Entschlüsselungseinheit (120) umfasst, wobei die Verschlüsselungs-/Entschlüsselungseinheit (120) zum Verschlüsseln der Konfigurationsdaten und zum Entschlüsseln der Verneblungsdaten konfiguriert ist.

10. Medizinische Auswertevorrichtung (10) nach einem der Ansprüche 1 bis 9, wobei die Auswerteeinheit (110) ferner konfiguriert ist, um eine tägliche Einhaltungsrate und/oder eine kumulative Einhaltungsrate für einen definierten Zeitraum des Therapieprotokolls zu berechnen.

11. Medizinische Auswertevorrichtung (10) nach einem der Ansprüche 1 bis 10, wobei die Auswerteeinheit (110) ferner konfiguriert ist, um einen Einhaltungsbericht für jeden Benutzer oder eine definierte Benutzergruppe zu erzeugen.

12. Medizinische Auswertevorrichtung (10) nach einem der Ansprüche 1 bis 11, wobei die Verneblungsdaten jeweilige Verneblungsstartzeiten und Verneblungsdauern für aufeinanderfolgende Betriebe des Aerosolverneblers (30) beinhalten, und wobei die Auswerteeinheit (110) ferner konfiguriert ist, um zu bestimmen, ob für die aufeinanderfolgenden Betriebe des Aerosolverneblers (30) jeweils eine erforderliche Mindestinhalationsdauer erreicht wird.

13. Medizinische Auswertevorrichtung (10) nach einem der Ansprüche 1 bis 12, wobei die Auswerteeinheit (110) ferner konfiguriert ist, um die therapiebezogenen Rückmeldedaten zu erzeugen, wenn die tägliche Einhaltungsrate und/oder die kumulative Einhaltungsrate nicht mit den jeweiligen im Therapieprotokoll definierten Parametern übereinstimmen.

14. Medizinische Auswertevorrichtung (10) nach einem der Ansprüche 1 bis 13, wobei die therapiebezogenen Rückmeldedaten konfiguriert sind, um eine Indikation an der Verneblersteuervorrichtung (40) zu erzeugen.

15. Medizinische Auswertevorrichtung (10) nach einem der Ansprüche 1 bis 14, wobei die Auswerteeinheit (110) ferner konfiguriert ist, um die Einhaltung des Therapieprotokoll medikamentenspezifisch zu bewerten.

16. Medizinische Auswertevorrichtung (10) nach einem der Ansprüche 1 bis 15, wobei die Diagnosevorrichtung (50) eine Spirometrievorrichtung und/oder eine Vorrichtung zum Messen von Nicht-Atmungs-Parametern ist.

17. Medizinische Auswertevorrichtung (10) nach Anspruch 1, wobei die zweiten Konfigurationsdaten mindestens einen von Atemwerten und Nicht-Atemwerten umfassen.

18. Medizinische Auswertevorrichtung (10) nach Anspruch 17, wobei die Atemwerte Atmungsdaten, insbesondere Atemfrequenz, einen Exhalations- oder Inhalationsstrom, Inhalations-/Exhalationsdauer und/oder ein Inhalationsvolumen beinhalten.

19. Medizinische Auswertevorrichtung (10) nach einem der Ansprüche 1, 17 oder 18, wobei die medizinische Auswertevorrichtung (10) ferner konfiguriert ist, um die Diagnosedaten beim Anpassen der ersten Konfigurationsdaten für die Verneblersteuervorrichtung zu verwenden.

20. Computerprogramm mit Anweisungen, die konfiguriert sind, um bei Ausführung auf einem oder mehreren Datenprozessoren, die über eine Kommunikationsvorrichtung mit einer Verneblersteuervorrichtung, einem Aerosolvernebler und einer Diagnosevorrichtung interagieren, den Datenprozessor oder die mehreren Datenprozessoren zu veranlassen, eine medizinische Auswertevorrichtung (10) nach einem der Ansprüche 1 bis 19 zu implementieren.

21. System, umfassend:

eine medizinische Auswertevorrichtung (10) nach einem der Ansprüche 1 bis 19;
eine Kommunikationsvorrichtung (20);
eine Verneblersteuervorrichtung (40); und
einen Aerosolvernebler (30), ferner umfassend die Diagnosevorrichtung (50).

## Revendications

1. Dispositif d'évaluation médicale (10) pour évaluer une adhésion à un protocole thérapeutique en utilisant un nébuliseur d'aérosol (30) présentant un générateur d'aérosol (31) pour nébuliser un médicament liquide selon ledit protocole thérapeutique, ledit dispositif d'évaluation médicale (10) comprenant :

une unité de communication (100), configurée pour établir une connexion de communication avec un dispositif de communication (20), ladite connexion de communication étant configurée pour :

- fournir des premières données de configuration à un dispositif de commande de nébuliseur (40) dudit nébuliseur d'aérosol via ledit dispositif de communication (20), lesdites premières données de configuration servant à configurer ledit nébuliseur d'aérosol selon ledit protocole thérapeutique ;
- recevoir des données de nébulisation indiquant une mise en œuvre dudit nébuliseur d'aérosol en provenance dudit dispositif de communication (20) ;

une unité d'évaluation (110), configurée pour évaluer ladite adhésion audit protocole thérapeutique sur la base d'une comparaison desdites données de nébulisation reçues à des paramètres respectifs définis à l'intérieur dudit protocole thérapeutique ;
dans lequel ladite unité d'évaluation (110) est en outre configurée pour produire des données de rétroaction liées à la thérapie, dans lequel
ledit dispositif d'évaluation médicale (10) est en outre conçu pour évaluer l'adhésion audit protocole thérapeutique en utilisant de plus un dispositif de diagnostic (50) pour surveiller un état des voies respiratoires et/ou un état et/ou une fonction des poumons du patient,
dans lequel ladite connexion de communication est en outre établie pour :

- fournir des secondes données de configuration pour un dispositif de commande de diagnostic dudit dispositif de diagnostic (50) via ledit dispositif de communication (20), lesdites secondes données de configuration servant à configurer ledit dispositif de diagnostic (50) selon ledit protocole thérapeutique ; et

- recevoir des données de diagnostic indiquant ledit état des voies respiratoires et/ou ledit état et/ou ladite fonction des poumons du patient,

dans lequel ledit dispositif d'évaluation médicale (10) est en outre configuré pour utiliser lesdites données de diagnostic en adaptant ledit protocole thérapeutique.

**2.** Dispositif d'évaluation médicale (10) selon la revendication 1,
dans lequel ladite unité de communication (100) est en outre configurée pour transmettre une association entre une valeur d'identification du dispositif de commande (40) et une valeur d'identification du dispositif de communication (20) au dispositif de communication (20).

**3.** Dispositif d'évaluation médicale (10) selon l'une des revendications 1 à 2,
dans lequel ladite unité de communication (100) est en outre configurée pour transmettre une instruction audit dispositif de communication (20) pour activer l'établissement d'une connexion de communication sans fil entre ledit dispositif de communication (20) et ledit dispositif de commande de nébuliseur (40).

**4.** Dispositif d'évaluation médicale (10) selon la revendication 3,
dans lequel ladite unité de communication (100) est en outre configurée pour transmettre une instruction de désactivation audit dispositif de communication (20) pour désactiver ladite connexion de communication sans fil entre ledit dispositif de communication (20) et ledit dispositif de commande de nébuliseur (40).

**5.** Dispositif d'évaluation médicale (10) selon l'une des revendications 1 à 4, dans lequel les premières données de configuration comprennent au moins une d'une valeur d'identification dudit dispositif de commande (40), une date courante, une heure courante, une durée maximale de nébulisation, un nombre maximal d'enregistrements pouvant être stockés, une désactivation du transfert de données automatique et une identification d'un ou plusieurs médicaments utilisables pour ledit médicament liquide.

**6.** Dispositif d'évaluation médicale selon l'une des revendications 1 à 5, dans lequel les données de nébulisation comprennent au moins l'une d'une date de nébulisation, d'une heure de nébulisation, d'une durée de nébulisation, d'un ou plusieurs critères d'interruption pour l'arrêt du nébuliseur d'aérosol, un état d'usage par un utilisateur, un niveau de remplissage du médicament liquide dans le réservoir, des caractéristiques de flux/volume/temps d'inhalation/exhalation dudit utilisateur et une liste d'un ou plusieurs médicaments utilisés pour ledit médicament liquide.

**7.** Dispositif d'évaluation médicale (10) selon l'une des revendications 1 à 6, dans lequel ladite réception desdites données de nébulisation est associée à la réception d'une identification dudit dispositif de communication (20) et d'une identification dudit dispositif de commande de nébuliseur (40).

**8.** Dispositif d'évaluation médicale (10) selon l'une des revendications 1 à 7, dans lequel ladite unité d'évaluation (110) est en outre configurée pour évaluer ladite adhésion seulement si ladite identification reçue du dispositif de communication (20) et ladite identification reçue du dispositif de commande de nébuliseur (40) coïncident avec un couple d'identifications stocké.

**9.** Dispositif d'évaluation médicale (10) selon l'une des revendications 1 à 8, comprenant en outre une unité de chiffrage/déchiffrage (120), ladite unité de chiffrage/déchiffrage (120) étant configurée pour chiffrer lesdites données de configuration et pour déchiffrer lesdites données de nébulisation.

**10.** Dispositif d'évaluation médicale (10) selon l'une des revendications 1 à 9, dans lequel ladite unité d'évaluation (110) est en outre configurée pour calculer un taux d'adhésion quotidien et/ou un taux d'adhésion cumulatif pendant une période de temps définie dudit protocole thérapeutique.

**11.** Dispositif d'évaluation médicale (10) selon l'une des revendications 1 à 10, dans lequel ladite unité d'évaluation (110) est en outre configurée pour produire un rapport d'adhésion pour chaque utilisateur ou un groupe d'utilisateurs défini.

**12.** Dispositif d'évaluation médicale (10) selon l'une des revendications 1 à 11, dans lequel lesdites données de nébulisation incluent des temps respectifs de début de nébulisation et des durées de nébulisation pour des mises en œuvre successives dudit nébuliseur d'aérosol (30), et dans lequel ladite unité d'évaluation (110) est en outre configurée pour déterminer si une durée d'inhalation minimale exigée est respectivement atteinte pour les mises en œuvre successives dudit nébuliseur d'aérosol (30).

13. Dispositif d'évaluation médicale (10) selon l'une des revendications 1 à 12, dans lequel ladite unité d'évaluation (110) est en outre configurée pour produire lesdites données de rétroaction liées à la thérapie si ledit taux d'adhésion quotidien et/ou ledit taux d'adhésion cumulatif ne correspondent pas aux paramètres respectifs définis dans le protocole thérapeutique.

14. Dispositif d'évaluation médicale (10) selon l'une des revendications 1 à 13, dans lequel lesdites données de rétroaction liées à la thérapie sont configurées pour produire une indication au niveau du dispositif de commande de nébuliseur (40).

15. Dispositif d'évaluation médicale (10) selon l'une des revendications 1 à 14, dans lequel ladite unité d'évaluation (110) est en outre configurée pour évaluer de manière spécifique au médicament ladite adhésion audit protocole thérapeutique.

16. Dispositif d'évaluation médicale (10) selon l'une des revendications 1 à 15, dans lequel ledit dispositif de diagnostic (50) est un dispositif de spirométrie et/ou un dispositif pour mesurer des paramètres non respiratoires.

17. Dispositif d'évaluation médicale (10) selon la revendication 1, dans lequel les secondes données de configuration comprennent au moins une de valeurs respiratoires et de valeurs non respiratoires.

18. Dispositif d'évaluation médicale (10) selon la revendication 17, dans lequel les valeurs respiratoires incluent des données respiratoires, particulièrement une fréquence respiratoire, un flux d'exhalation ou d'inhalation, une durée d'inhalation/exhalation et/ou un volume d'inhalation.

19. Dispositif d'évaluation médicale (10) selon l'une des revendications 1, 17, ou 18, dans lequel ledit dispositif d'évaluation médicale (10) est en outre configuré pour utiliser lesdites données de diagnostic en adaptant lesdites premières données de configuration pour ledit dispositif de commande de nébuliseur.

20. Programme informatique incluant des instructions configurées, lorsqu'elles sont exécutées sur un ou une pluralité de processeurs de données interagissant via un dispositif de communication avec un dispositif de commande de nébuliseur, un nébuliseur d'aérosol et un dispositif de diagnostic, pour amener le processeur de données ou la pluralité de processeurs de données à mettre en œuvre un dispositif d'évaluation médicale (10) selon l'une quelconque des revendications 1 à 19.

21. Système, comprenant :

un dispositif d'évaluation médicale (10) selon l'une quelconque des revendications 1 à 19 ;
un dispositif de communication (20) ;
un dispositif de commande de nébuliseur (40) ; et
un nébuliseur d'aérosol (30), comprenant en outre ledit dispositif de diagnostic (50).

Fig. 1
PRIOR ART

Fig. 2

| identification value of control device 40 | identification value of communication device 20 | type of communication connection |
|---|---|---|
| MAC address $40_{MAC}$ Bluetooth name $40_{BT}$ serial number $40_{SN}$ | MAC address $20_{MAC}$ Bluetooth name$20_{BT}$ serial number $20_{SN}$ | Bluetooth |
| ... | ... | Wi-Fi |
| ... | ... | ... |

Fig. 3a

| identification value of control device 40 | identification value of communication device 20 | type of communication connection | user identification |
|---|---|---|---|
| MAC address $40_{MAC}$ Bluetooth name $40_{BT}$ serial number $40_{SN}$ | MAC address $20_{MAC}$ Bluetooth name $20_{BT}$ serial number $20_{SN}$ | Bluetooth | user A |
| ... | ... | Wi-Fi | user A |
| ... | ... | ... | ... |

Fig. 3b

Fig. 4

# Inhalation Data Report

Study: 121 Huck Test
Drug Name: text drug

Max. time/therapy(min): 08:00
Duration of Study (days): 360
Therapies per day: 2

Patient-No.:  0000121
Card-No.:  2

| Start Date/Time | End Date/Time | Duration (min) | Day of Therapy | Status |
|---|---|---|---|---|
| 26.02.2009 09:20:00 | 26.02.2009 09:30:00 | 2.00 | 1 | Inhalation finished |
| 26.02.2009 13:19:33 | 26.02.2009 13:22:41 | 3.13 | 1 | Inhalation finished |
| 27.02.2009 08:30:00 | 27.02.2009 08:32:00 | 2.00 | 2 | Inhalation finished |
| 27.02.2009 09:20:00 | 27.02.2009 09:30:00 | 2.00 | 2 | Inhalation finished |
| 28.02.2009 08:30:00 | 28.02.2009 08:32:00 | 2.00 | 3 | Inhalation finished |
| 28.02.2009 09:20:00 | 28.02.2009 09:30:00 | 0.83 | 3 | Inhalation finished |
| 01.03.2009 08:30:00 | 01.03.2009 08:32:00 | 2.00 | 4 | Inhalation finished |
| 01.03.2009 09:20:00 | 01.03.2009 09:30:00 | 2.00 | 4 | Inhalation finished |
| 02.03.2009 08:30:00 | 02.03.2009 08:32:00 | 2.00 | 5 | Inhalation finished |
| 02.03.2009 09:20:00 | 02.03.2009 09:30:00 | 2.00 | 5 | Inhalation finished |
| 03.03.2009 08:30:00 | 03.03.2009 08:32:00 | 2.00 | 6 | Inhalation finished |
| 04.03.2009 08:30:00 | 04.03.2009 08:32:00 | 2.00 | 7 | Inhalation finished |
| 04.03.2009 09:20:00 | 04.03.2009 09:30:00 | 2.00 | 7 | Inhalation finished |
| 05.03.2009 08:30:00 | 05.03.2009 08:32:00 | 2.00 | 8 | Inhalation finished |
| 05.03.2009 09:20:00 | 05.03.2009 09:30:00 | 2.00 | 8 | Inhalation finished |
| 06.03.2009 08:30:00 | 06.03.2009 08:32:00 | 2.00 | 9 | Inhalation finished |

**Fig. 5**

EP 3 203 397 B1

EP 3 203 397 B1

Fig. 6

# Compliance Report Per Card

PARI Pharma
Advancing Aerosol Therapies

Study: 161    Studie

Period:    11.03.2008 TO 07.04.2008
Patient:   00020         Card No.:   24

## Compliance per Day %/Cumulative Compliances %

Day of Therapy

Final compliance of this report  86%

# Fig. 7

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10243371 A1
- US 20060237001 A1
- US 2006237002 A2
- US 2015174349 A1